# FASCICULE DE BREVET EUROPEEN

(11) **EP 3 389 681 B1**
(45) Date de publication et mention de la délivrance du brevet: **05.02.2020**
(21) Numéro de dépôt: 16812752.0
(22) Date de dépôt: 16.12.2016
(51) Int. Cl.: A61K 35/741, A61K 47/26, A61K 9/16, A61K 9/19, C12N 1/04, A61P 1/00, A61P 31/00

(54) **COMPOSITION LYOPHILISEE POUR LA CONSERVATION DE MICROBIOTE DANS SON ECOSYSTEME**
LYOPHILISIERTE ZUSAMMENSETZUNG ZUR KONSERVIERUNG VON BIOZÖNOSE IN IHREM ÖKOSYSTEM
LYOPHILIZED COMPOSITION FOR PRESERVING MICROBIOTA IN ITS ECOSYSTEM

(30) Priorité: 18.12.2015 FR 1562836
(43) Date de publication de la demande: 24.10.2018
(73) Titulaire: Institut National De La Recherche Agronomique, 75007 Paris (FR); Université Paris Descartes, 75006 Paris (FR); Université Paris Diderot - Paris 7, 75205 Paris Cedex 13 (FR); Assistance Publique - Hôpitaux de Paris, 75004 Paris (FR)
(72) Inventeur: KAPEL, Nathalie, 75015 Paris (FR); WALIGORA-DUPRIET, Anne-Judith, 92140 Clamart (FR); THOMAS, Muriel, 91430 Igny (FR); CHARRUEAU, Christine, 91200 Athis-Mons (FR); JOLY, Francisca, 75017 Paris (FR); MAYEUR, Camille, 78330 Fontenay-le-Fleury (FR); ROBERT, Véronique, 91470 Limours en Hurepoix (FR); DELANNOY, Johanne, 95800 Cergy-le-Haut (FR)
(74) Mandataire: Gevers & Orès
(86) Numéro de dépôt international: PCT/EP2016/081603
(87) Numéro de publication internationale: WO 2017/103225

(56) Documents cités:
- WO-A1-2008/035332
- WO-A1-2013/142792
- WO-A1-2014/152484
- WO-A1-2016/170285
- WO-A2-2007/070052
- WO-A2-2012/016287
- SEMYONOV D ET AL: "Microencapsulation of Lactobacillus paracasei by spray freeze drying", FOOD RESEARCH INTERNATIONAL, ELSEVIER APPLIED SCIENCE, BARKING, GB, vol. 43, no. 1, 1 janvier 2010 (2010-01-01), pages 193-202, XP026813715, ISSN: 0963-9969 [extrait le 2009-09-27]
- TIAN HONGLIANG ET AL: "Freeze-dried, Capsulized Fecal Microbiota Transplantation for Relapsing Clostridium difficile Infection", JOURNAL OF CLINICAL GASTROENTEROLOGY UNITED STATES, USA, vol. 49, no. 6, 1 juillet 2015 (2015-07-01), pages 537-538, XP009189478, ISSN: 1539-2031, DOI: 10.1097/MCG.0000000000000330
- BORODY THOMAS J ET AL: "Fecal Microbiota Transplantation: Expanding Horizons for Clostridium difficile Infections and Beyond", ANTIBIOTICS,, vol. 4, no. 3, 1 janvier 2015 (2015-01-01) , pages 254-266, XP009189481,
- YOUNGSTER ILAN ET AL: "Oral, capsulized, frozen fecal microbiota transplantation for relapsing Clostridium difficile infection", JAMA : THE JOURNAL OF THE AMERICAN MEDICAL ASSOCIATION, AMERICAN MEDICAL ASSOCIATION, UNITED STATES, vol. 312, no. 17, 5 novembre 2014 (2014-11-05), pages 1772-1778, XP009189475, ISSN: 1538-3598, DOI: 10.1001/JAMA.2014.13875
- LIN ENMOORE ET AL: "Twelve Week Storage Trial of Microbial Viability in Lyophilized and Frozen Fecal Microbiota Preparations", GASTROENTEROLOGY, ELSEVIER, PHILADELPHIA, PA, vol. 148, no. 4, Suppl.1, 1 mai 2014 (2014-05-01), page S962, XP009189480, ISSN: 0016-5085

## Description

La présente invention a trait au domaine de la conservation sous forme d'un lyophilisat d'un microbiote dans son écosystème (MDSE). Plus particulièrement, elle concerne une composition lyophilisée de selles permettant la conservation jusqu'à plusieurs mois du microbiote fécal en vue d'une transplantation chez un individu. L'invention concerne également l'utilisation thérapeutique chez l'homme ou l'animal d'une telle composition, notamment sous forme de gélules pour une administration par voie orale.

De nombreuses études montrent l'intérêt de la transplantation de microbiote fécal (TMF) pour la prise en charge thérapeutique de certaines pathologies impliquant un déséquilibre du microbiote fécal au sein de son écosystème, appelé dysbiose, telles que par exemple les infections *à Clostridium difficile* et leurs récidives (Van Nood E. *et al.,* 2013). La TMF dispose déjà d'un niveau de preuve clinique élevé de sorte que le recours à cette technique pour cette indication figure dans les plus récentes recommandations, tant européennes (Debast SB. *et al.,* 2014) que nord-américaines (Surawicz CM. *et al.,* 2013). L'application de cette technique à d'autres contextes pathologiques de dysbiose, comme les maladies inflammatoires de l'intestin, le syndrome du côlon irritable, le syndrome métabolique, l'obésité, le diabète, les bactéries multirésistantes, voire les troubles du développement neurologique, dont les étiologies sont incertaines ou inconnues, est également envisagée (Vrieze A. *et al.,* 2013, Kelly CR. *et al.,* 2015).

Malgré un engouement très fort depuis quelques années de la part de la communauté médicale et l'émergence récente de demandes des patients, il n'existe pas aujourd'hui de procédure standardisée pour la réalisation de la TMF, que ce soit pour la sélection du donneur, le mode de préparation des selles ou la voie d'administration.

D'une manière générale, le donneur est sélectionné sur la base d'un examen clinique et du dépistage d'un panel important d'agents pathogènes comme les bactéries, les virus ou les parasites, réalisés en amont du don ; il en résulte un taux de validation des donneurs potentiels inférieurs à 20% (Borody T. *et al.,* 2015). Ceci, ajouté au délai d'obtention des résultats de la recherche d'agents pathogènes, limite fortement la réalisation de dons exploitables.

Les modalités d'administration d'une TMF sont diverses. Le microbiote fécal peut être administré par sonde nasogastrique ou nasoduodénale, au cours d'une coloscopie ou encore par lavement rectal (Kelly CR. *et al.,* 2015), chacune de ces méthodes présentant ses propres avantages et inconvénients. La voie nasogastrique ou nasoduodénale, par exemple, peut être mal perçue par l'individu à traiter, avec un risque de vomissement, voire d'aspiration et peut nécessiter un contrôle radiologique lors de la mise en place de la sonde. La voie colique présente l'intérêt de permettre l'observation de la muqueuse en même temps que l'administration mais elle présente des facteurs de morbidité et un coût significatifs. Enfin, la voie du lavement rectal est la plus facile à mettre en oeuvre et la moins coûteuse mais il peut être difficile pour l'individu à traiter d'assurer la rétention du matériel administré. A ces difficultés s'ajoutent des réticences de la part des patients liées aux caractères organoleptiques de la préparation.

Les recommandations actuelles du Groupe Français de Transplantation Fécale dans le cadre des colites à *Clostridium difficile* (Sokol H. *et al.,* 2015) et de l'ANSM pour les essais cliniques (ANSM, 2015) portent sur une administration dans les 6h suivant l'émission des selles. Pour faciliter l'accès au don, des préparations fécales conservées congelées à -80°C et donc immédiatement disponibles sont proposées. Leur efficacité est similaire à celle observée pour les transplantations utilisant du matériel frais (Hamilton MJ. *et al.,* 2012 ; Youngster I. *et al.,* 2014a ; Lee *et al.,* 2016). Ces préparations congelées nécessitent la réalisation d'une dilution au sein d'une solution contenant un cryoprotecteur pour protéger le microbiote des effets de la congélation. Le seul cryoprotecteur décrit dans la littérature pour la congélation des selles en vue de leur transplantation est le glycérol à la concentration de 10%. L'efficacité de la transplantation réalisée avec ces préparations congelées contenant du glycérol est maintenue jusqu'à 5 mois post-préparation (Younster I. *et al.,* 2014a).

L'administration *per os* de gélules remplies avec ce matériel et conservées à -80°C a été rapportée avec des résultats satisfaisants (Youngster I, *et al.,* 2014b; Younster *et al.,* 2016). Ce type de préparation ouvre donc de nouvelles perspectives pour une approche galénique facilitant l'administration chez l'individu à traiter, en particulier le patient. Cependant, en l'état actuel, elle implique une production encore très empirique et nécessite une conservation à -80°C à laquelle sont liés des problèmes de stockage.

Il existe donc un besoin non satisfait de disposer de préparations de selles dont la préparation et le stockage seraient facilités tout en maintenant les qualités initiales du microbiote fécal pendant la durée de conservation et qui seraient disponibles sous une forme galénique permettant l'administration par une voie d'abord aisée et non invasive d'un volume réduit.

Dans ce contexte, les inventeurs ont maintenant développé une composition lyophilisée comprenant des selles diluées dans un mélange optimisé de cryoprotecteurs dans le but de faciliter la production de préparations fécales, leur disponibilité et leur conservation à long terme, tout en améliorant l'acceptabilité et le confort des individus à traiter.

La lyophilisation est un procédé de conservation utilisé dans l'industrie pharmaceutique pour la préservation des souches bactériennes. La lyophilisation doit être réalisée dans des conditions optimales pour assurer le maintien de la viabilité ainsi que l'équilibre des différents genres bactériens, notamment dans le cas de mélanges complexes comme c'est le cas pour le MDSE en général et les selles en particulier. L'utilisation de cryoprotecteurs appropriés permet d'assurer la survie complète ou au moins partielle, des bactéries lors d'une congélation.

On distingue trois catégories de molécules cryoprotectrices vis-à-vis des organismes vivants, notamment des bactéries :
- les molécules de haute masse moléculaire demeurant en milieu extracellulaire, telles que les protéines et les polysaccharides, qui facilitent l'efflux aqueux à partir des cellules pendant la phase de congélation, limitant ainsi la formation de cristaux de glace intracellulaires. En augmentant la viscosité du milieu extracellulaire, ces molécules réduisent également la croissance cristalline ;
- les molécules capables de traverser la paroi cellulaire comme les acides aminés et les oligosaccharides ;
- les molécules pénétrant la paroi cellulaire et la membrane cytoplasmique telles le DMSO et le glycérol (Carvalho A.S. *et al.,* 2004).

Les agents pénétrants des seconde et troisième catégories limitent la déshydratation cellulaire et abaissent la température de congélation à l'intérieur de la cellule, réduisant ainsi la concentration saline et diminuant le choc osmotique (Béal C. *et al.,* 2015).

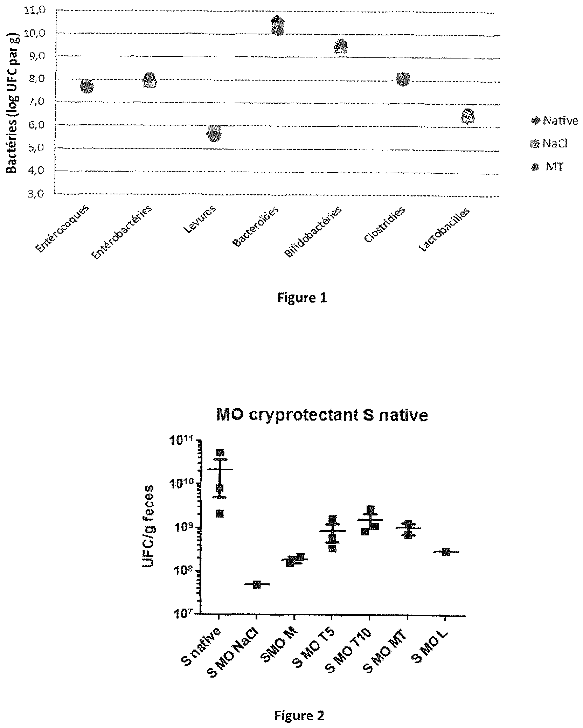

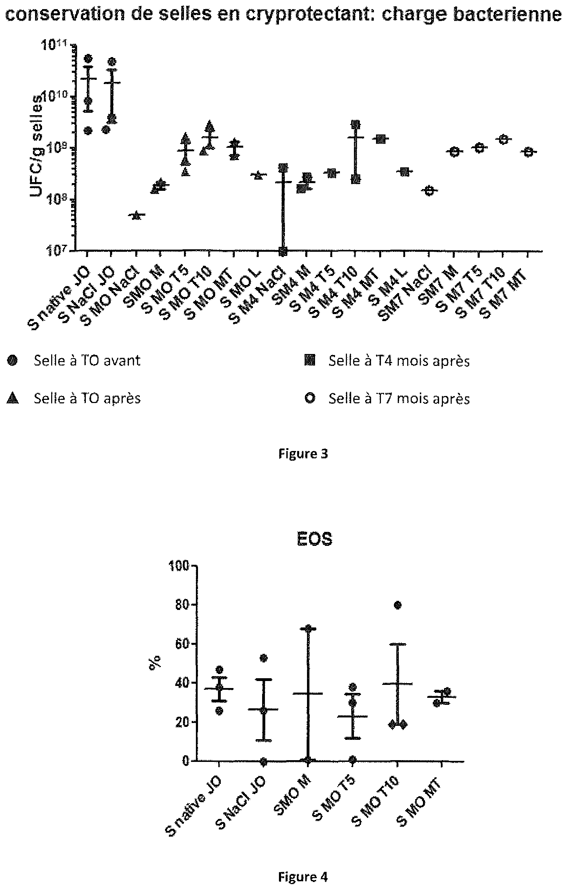

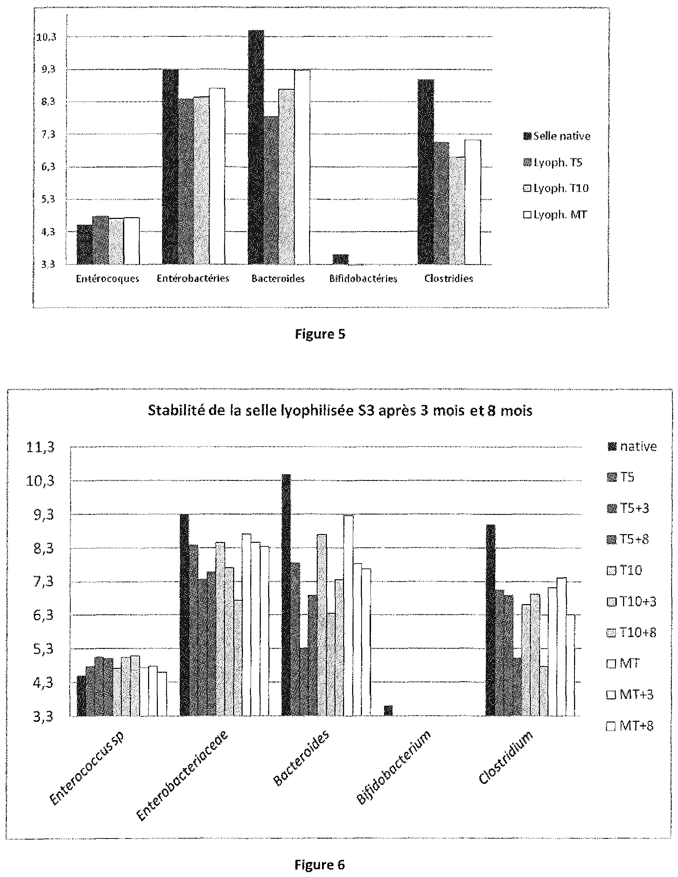

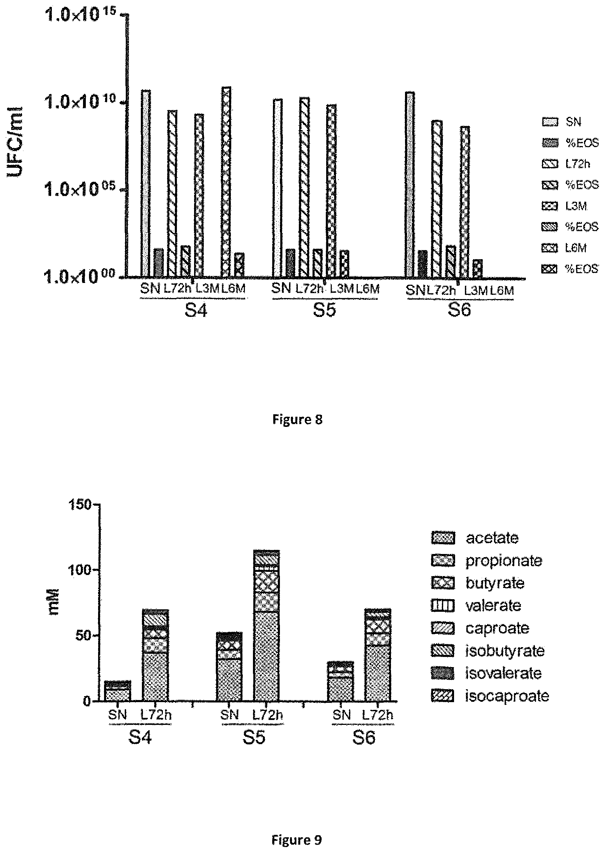

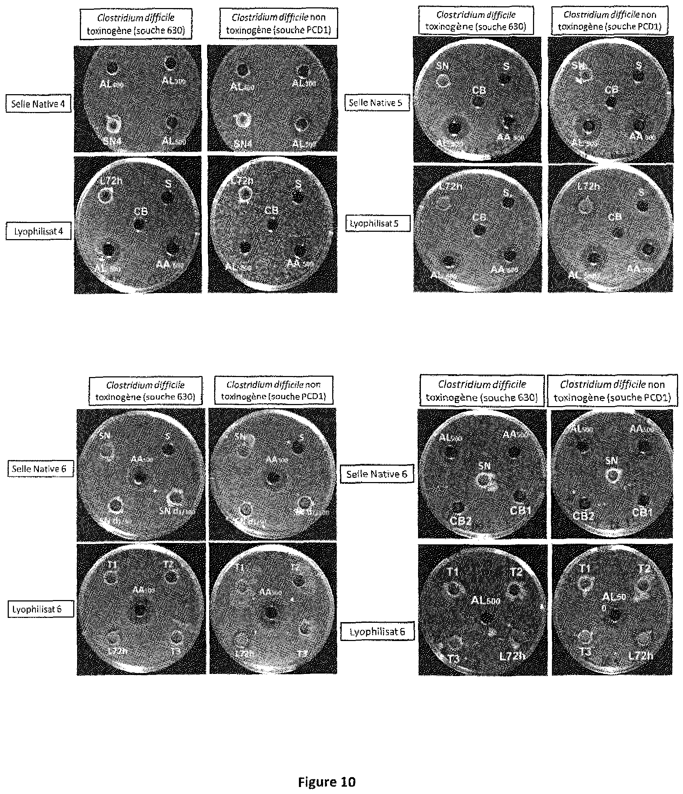

Afin d'optimiser la conservation des organismes vivants constitutifs du microbiote fécal, les inventeurs ont évalué plusieurs cryoprotecteurs, isolés ou en association. Le glycérol à 10% classiquement utilisé pour les TMF réalisées avec du matériel congelé (Hamilton MJ. *et al.,* 2012 ; Youngster I. *et al.,* 2014a) apparait non adapté à la réalisation de lyophilisat. Parmi les cryoprotecteurs testés, deux se sont révélés prometteurs : la maltodextrine et le tréhalose. Si le tréhalose donne des résultats performants, les résultats obtenus avec un mélange de maltodextrine et de tréhalose sont encore meilleurs aussi bien pour le maintien de la viabilité du microbiote (Figures 1 et 2) que pour le maintien de sa diversité (Figure 5). Le maintien de la diversité est en particulier très important du fait que la TMF a justement pour but de rétablir l'équilibre entre les différentes populations présentes dans l'écosystème intestinal de l'individu bénéficiaire. Les effets protecteurs du mélange de maltodextrine et de tréhalose vis-à-vis du microbiote sont observés pendant au moins 3 mois et jusqu'à 7 mois après lyophilisation (Figures 3, 6 et 7). Le mélange de maltodextrine et de tréhalose est également plus favorable au maintien de la viabilité des bactéries extrêmement sensibles à l'oxygène (EOS) telles que les bactéries du groupe *Clostridium leptum* (Figures 4 et 8). De plus, la concentration de métabolites bactériens tels les acides gras à chaines courtes représentatifs des capacités fonctionnelles du microbiote fécal est préservée et même augmentée au sein du lyophilisat comparativement à la selle native (Figure 9). Enfin, une activité anti-*Clostridium difficile* directe des lyophilisats est mise en évidence *in vitro* avec inhibition de la croissance bactérienne de 2 souches de *Clostridium difficile,* l'une toxinogène (souche 630) et l'autre non toxinogène (souche PCD1) (Figure 10).

L'objet de la présente invention est défini par les revendications 1-10.

Ainsi, la description concerne l'utilisation d'un mélange de cryoprotecteurs pour la conservation d'un microbiote dans son écosystème (MDSE) sous forme lyophilisée, caractérisée en ce que ledit mélange de cryoprotecteurs comprend de la maltodextrine (M) et du tréhalose (T) dans un rapport pondéral M/T compris entre 35/65 et 45/55.

La maltodextrine est un cryoprotecteur polysaccharidique non pénétrant dans les parois cellulaires tandis que le tréhalose est un cryoprotecteur oligosaccharidique pénétrant les parois cellulaires.

Par « microbiote dans son écosystème » ou MDSE, on entend un microbiote dont la composition est non modifiée par rapport à celle existant à l'état naturel dans l'organisme. Le microbiote et son écosystème constituent un mélange complexe et riche, constitué notamment de bactéries, champignons, virus, bactériophages mais aussi de débris cellulaires, de nombreux peptides et protéines sécrétés par les entérocytes et cellules de la *lamina proprio* avec leurs produits d'excrétion/sécrétion tels les IgA sécrétoires, la calprotectine, les cytokines et autres peptides anti-inflammatoires ou antimicrobiens type défensines, de produits bactériens sécrétés ou non, incluant des métabolites tels que les acides gras à chaîne courte, les bactériocines, et toutes les autres molécules biologiquement actives produites par les bactéries ou d'autres microorganismes du microbiote, de mucus, de composés glucidiques type mono et polysaccharides, d'oligoéléments. Le MDSE constitue donc une matrice complexe que l'on peut qualifier de vivante, dans laquelle de nombreuses interactions moléculaires et cellulaires se produisent.

Les MDSE peuvent être conservés sous forme lyophilisée en utilisant un mélange de maltodextrine et de tréhalose selon la description. A titre d'exemples, les MDSE pouvant être conservés selon cette méthode sont le microbiote intestinal, anciennement appelé flore intestinale, le microbiote fécal contenu dans les selles et le microbiote vaginal anciennement appelé flore vaginale. Chacun de ces microbiotes est caractérisé par des groupes de bactéries dominantes bien connus de l'homme du métier.

Ainsi, l'invention concerne l'utilisation d'un mélange de cryoprotecteurs pour la conservation d'un microbiote dans son écosystème sous forme lyophilisée, caractérisée en ce que ledit mélange de cryoprotecteurs comprend de la maltodextrine (M) et du tréhalose (T) dans un rapport pondéral M/T compris entre 35/65 et 45/55, le microbiote dans son écosystème étant le microbiote intestinal, le microbiote fécal ou le microbiote vaginal.

Dans un mode de réalisation préféré, le MDSE à conserver consiste en des selles.

Dans un mode de réalisation, l'invention concerne l'utilisation d'un mélange de cryoprotecteurs telle que décrite précédemment, dans laquelle le microbiote dans son écosystème consiste en des selles.

Par « lyophilisation », on entend, au sens de la description, le procédé consistant à éliminer l'eau d'un produit à l'aide de la congélation puis d'une évaporation sous vide de la glace sans passage par l'état liquide, appelée sublimation. Cette technique est bien connue de l'homme du métier. On distingue trois phases majeures dans un cycle de lyophilisation :
- la congélation, où les produits sont réfrigérés à des températures de l'ordre de -20 °C à -80 °C ; l'eau se transforme alors en glace ;
- la dessiccation primaire, sous vide, qui consiste à sublimer la glace libre interstitielle ;
- la dessiccation secondaire, qui permet d'extraire par désorption les molécules d'eau piégées à la surface des produits séchés.

Par « selles », on entend, au sens de la description, les matières obtenues après les phases de digestion et d'absorption des aliments et leur transit dans le tractus intestinal. Elles sont normalement constituées d'eau à 75-85% en poids et de matières sèches à 15-25% en poids [cellules intestinales desquamées et cellules immunitaires issues de la *lamina propria* avec leurs produits d'excrétion/sécrétion à type d'IgA sécrétoires, calprotectine, cytokines et autres peptides anti-inflammatoires ou antimicrobiens..., éléments constitutifs de microbiote (bactéries, virus, champignons, bactériophages) et les produits bactériens sécrétés ou non, incluant des métabolites tels que les acides gras à chaîne courte, les bactériocines, et toutes les autres molécules biologiquement actives produites par les bactéries ou d'autres microorganismes du microbiote ; mucus ; débris cellulaires dont les fibres et la cellulose qui proviennent de la partie non digestible des végétaux, lipides, fer, oligoéléments...]. Les selles représentent, au sens de la description, des selles fraîchement produites par un individu en bonne santé. La sélection des donneurs chez les humains et la préparation de selles peuvent être réalisées selon le protocole et les conditions définis dans les recommandations du Groupe Français de Transplantation Fécale (Sokol *et al.,* 2015), dans l'article intitulé « Practical implementation of faecal transplantation » de Kapel N. et al., 2014 et dans les recommandations de l'ANSM (2015).

Dans le mélange de cryoprotecteurs utilisé pour conserver sous forme lyophilisée selon la description, la maltodextrine (M) est utilisée avec le tréhalose (T) dans un rapport pondéral M/T compris entre 35/65 et 45/55, et de manière préférée de 40/60.

Selon un mode de réalisation préféré de la description, les cryoprotecteurs présents dans le mélange sont exclusivement la maltodextrine et le tréhalose.

Selon un autre mode de réalisation de la description, les cryoprotecteurs présents dans le mélange incluent la maltodextrine et le tréhalose ainsi qu'au moins un autre cryoprotecteur choisi parmi le mannitol, le sorbitol, le fructose, le glucose, le maltose, le saccharose, la gélatine, l'amidon, le sérum de veau fœtal et le lait.

Un autre objet de la description concerne une composition lyophilisée comprenant un microbiote dans son écosystème (MDSE) et un mélange de cryoprotecteurs, ledit mélange de cryoprotecteurs comprenant de la maltodextrine (M) et du tréhalose (T), utilisés dans un rapport pondéral M/T compris entre 35/65 et 45/55, et de manière préférée dans un rapport pondéral de 40/60.

Dans un mode de réalisation, l'invention concerne une composition lyophilisée comprenant un microbiote dans son écosystème et un mélange de cryoprotecteurs, caractérisée en ce que ledit mélange de cryoprotecteurs comprend de la maltodextrine et du tréhalose, utilisés dans un rapport pondéral M/T compris entre 35/65 et 45/55, le microbiote dans son écosystème étant le microbiote intestinal, le microbiote fécal ou le microbiote vaginal.

Dans la composition de la présente description, la maltodextrine est utilisée avec le tréhalose dans un rapport pondéral M/T compris entre 35/65 et 45/55, et de manière préférée dans un rapport pondéral de 40/60.

Selon un mode de réalisation préféré de la description, les cryoprotecteurs présents dans la composition lyophilisée sont exclusivement la maltodextrine et le tréhalose.

Selon un autre mode de réalisation de la description, les cryoprotecteurs présents dans la composition lyophilisée incluent la maltodextrine et le tréhalose ainsi qu'au moins un autre cryoprotecteur choisi parmi le mannitol, le sorbitol, le fructose, le glucose, le maltose, le saccharose, la gélatine, l'amidon, le sérum de veau fœtal et le lait.

La composition lyophilisée selon la description contient un mélange de MDSE et de cryoprotecteurs en une teneur pondérale de 15 à 35% et de 85 à 65% de la composition lyophilisée respectivement, de préférence en une teneur pondérale de 18 à 28% et de 82 à 72%. Ces teneurs pondérales peuvent varier en fonction de différents paramètres, notamment le degré d'hydratation de l'échantillon de MDSE ainsi que sa teneur en lipides, en fibres et en débris cellulaires qui peuvent varier, notamment dans le cas des selles. L'homme du métier saura adapter ces teneurs pondérales afin d'optimiser la composition à lyophiliser.

Ainsi, les inventeurs ont mis en évidence que la lyophilisation d'une composition comprenant des selles et un mélange de cryoprotecteurs, comprenant de la maltodextrine et du tréhalose, permet de conserver la viabilité et la biodiversité du microbiote fécal sur une période prolongée d'au moins 6 mois. Ils ont également mis en évidence une préservation de la production de métabolites bactériens dans les lyophilisats. En outre, ils ont montré la capacité des lyophilisats à d'inhiber le développement de *Clostridium difficile in vitro.*

Par « viabilité du microbiote fécal», on entend, au sens de la description, la part du microbiote fécal qui est vivant.

La « biodiversité du microbiote fécal » est représentée par les différentes populations microbiennes présentes dans le MDSE. Le maintien des populations constituantes peut être évalué par la stabilité de groupes de micro-organismes représentatifs au sein du microbiote.

Dans un mode de réalisation préféré, la présente description a pour objet une composition lyophilisée comprenant des selles et un mélange de cryoprotecteurs comprenant la maltodextrine (M) et le tréhalose (T) avec un rapport pondéral M/T compris entre 35/65 et 45/55, de manière préférée dans un rapport pondéral de 40/60.

Dans ce mode de réalisation préféré dans lequel le MDSE consiste en des selles, la quantité en poids de selles représente 15 à 35% de la composition lyophilisée et la quantité en poids du mélange de cryoprotecteurs représente 85 à 65% de la composition lyophilisée.

Dans un mode de réalisation encore davantage préféré dans lequel le MDSE consiste en des selles, la quantité en poids de selles représente 15 à 35% de la composition lyophilisée, la quantité en poids du mélange de cryoprotecteurs représente 85 à 65% de la composition lyophilisée et le mélange de cryoprotecteurs comprend de la maltodextrine (M) et du tréhalose (T) dans un rapport pondéral M/T compris entre 35/65 et 45/55, de manière préférée dans un rapport pondéral de 40/60.

Dans un mode de réalisation, l'invention concerne une composition lyophilisée comprenant un microbiote dans son écosystème et un mélange de cryoprotecteurs, caractérisée en ce que ledit mélange de cryoprotecteurs comprend de la maltodextrine et du tréhalose, utilisés dans un rapport pondéral M/T compris entre 35/65 et 45/55, dans laquelle(i) le microbiote dans son écosystème (MDSE) consiste en des selles, (ii) la quantité en poids de selles représente 15 à 35% de la composition lyophilisée et la quantité en poids du mélange de cryoprotecteurs représente 85 à 65% de la composition lyophilisée et (iii) la maltodextrine (M) et le tréhalose (T) sont utilisés dans un rapport pondéral M/T de 40/60.

En plus d'assurer le maintien de la charge bactérienne, de sa viabilité et de sa diversité en préservant l'équilibre entre les groupes bactériens dominants appartenant aux phylum des Firmicutes et des Bacteroidetes, la composition selon la description présente un avantage sérieux par rapport aux mélanges de souches bactériennes (Allen-Vercoe E. *et al.,* 2013). En effet, la composition de selles selon la description permet de conserver le microbiote fécal dans son écosystème naturel d'origine (écosystème fécal). Les interactions entre les différents constituants ainsi que les capacités fonctionnelles de cette matière complexe sont ainsi préservées et l'individu qui reçoit la TMF bénéficie alors des mêmes avantages que lors d'une transplantation de selles fraîches ou de l'administration d'un filtrat bactérien (Ott *et al.,* 2016).

Par "écosystème fécal", on entend, au sens de la description, tout ce qui compose les selles, c'est-à-dire, non seulement les bactéries, virus, champignons et bactériophages mais aussi les produits bactériens sécrétés ou non, incluant des métabolites tels que les acides gras à chaîne courte, les bactériocines, et toutes les autres molécules biologiquement actives produites par les bactéries ou d'autres microorganisme du microbiote ainsi que les cellules intestinales desquamées ou en apoptose (entérocytes, cellules immunitaires de la *lamina proprio)* et leurs produits d'excrétion/sécrétion (IgA sécrétoires, calprotectine, cytokines et autres peptides anti-inflammatoires ou antimicrobiens...), les éléments glucidiques mono- et polysaccharidiques tels les fibres et la cellulose qui proviennent de la partie non digestible des végétaux, résidus protéiques et lipidiques, oligoéléments, le mucus...

La composition de la description est particulièrement adaptée à une administration orale.

Elle peut, par exemple, se présenter sous la forme d'une gélule ou d'un comprimé gastro-résistant, de granulés ne nécessitant pas de réhydratation ou d'un spray, en fonction de l'individu à traiter.

Selon un mode de réalisation particulier de la description, la composition lyophilisée se présente sous forme d'une gélule ou d'un comprimé gastro-résistant. Cette présentation est particulièrement adaptée à une administration orale chez l'homme. Par « gélule ou comprimé gastro-résistant », on entend, au sens de la description, une forme galénique orale résistante aux sucs gastriques et entérosoluble.

Ces formulations peuvent en outre contenir un véhicule pharmaceutiquement acceptable afin de rendre la composition lyophilisée appropriée pour une administration chez le sujet à traiter. Ce véhicule est de préférence inerte, et dans tous les cas, de nature à maintenir la viabilité et la diversité du microbiote au sein de son écosystème et à libérer la composition lyophilisée au site approprié.

Ainsi, la présente invention a également pour objet une gélule gastro-résistante comprenant la composition lyophilisée telle que définie précédemment.

Le remplissage desdites gélules, qu'il soit manuel ou industriel, est réalisé volumétriquement. Pour cela il est nécessaire que le lyophilisat soit pulvérisé et que la poudre à répartir présente une bonne fluidité pour assurer un remplissage régulier des gélules (Le Hir et al., 2016).

Les propriétés d'écoulement d'une poudre peuvent être évaluées selon l'essai « 2.9.16. Ecoulement » de la Pharmacopée européenne (Pharmacopée Européenne., 2016) qui mesure le temps nécessaire à l'écoulement d'une prise d'essai à travers l'orifice d'un entonnoir de dimensions normalisées. Un temps inférieur ou égal à 10 secondes / 100 g de poudre correspond à un excellent écoulement. A l'opposé, le temps d'écoulement d'une poudre ne s'écoulant pas sera noté « infini ». Ceci est le cas d'un lyophilisat fécal brut qui ne peut donc pas être utilisé tel quel pour le remplissage de gélules.

Pour pallier cette limite, les inventeurs ont mis au point des gélules comprenant la composition lyophilisée telle que définie précédemment additionnée d'excipients d'écoulement tels que des diluants et des lubrifiants d'écoulement, capables d'améliorer la fluidité des lyophilisats fécaux.

Dans un mode de réalisation, l'invention concerne une gélule gastro-résistante telle que décrite précédemment, caractérisée en ce que ladite composition lyophilisée est mélangée à au moins un excipient d'écoulement.

Ces excipients d'écoulement utilisés dans la mise en gélules des lyophilisats de la description sont choisis parmi des excipients minéraux, tels que le phosphate dicalcique, des maltodextrines, le talc, une silice colloïdale hydrophile et une silice colloïdale hydrophobe.

Ainsi, selon la description, la gélule gastro-résistante comprend une composition lyophilisée telle que définie précédemment, mélangée à au moins un excipient d'écoulement choisi parmi un lubrifiant d'écoulement et/ou un diluant pour compression directe.

Selon un mode de réalisation particulier, l'excipient d'écoulement est un lubrifiant d'écoulement employé en une quantité conforme à l'usage classique ; de préférence, la quantité de lubrifiant mélangé au lyophilisat est comprise entre 0,1 et 1% en poids du mélange lyophilisat et lubrifiant, encore préférentiellement cette quantité de lubrifiant est de 0,5% en poids du mélange lyophilisat et lubrifiant.

Le lubrifiant d'écoulement peut être choisi parmi le talc, la silice colloïdale hydrophobe, la silice colloïdale hydrophile ; de préférence, il s'agit de la silice colloïdale hydrophobe.

Selon un autre mode de réalisation particulier, l'excipient d'écoulement est un diluant pour compression directe employé en une quantité conforme à l'usage classique ; de préférence, la quantité de diluant mélangé au lyophilisat est comprise entre 10 et 90% en poids du mélange lyophilisat et diluant, encore préférentiellement entre 15 et 75% ou 25 et 50% en poids du mélange lyophilisat et diluant.

Le diluant pour compression directe peut être choisi parmi le phosphate dicalcique ou les maltodextrines nébulisées.

Selon encore un autre mode de réalisation particulier, l'excipient d'écoulement comprend un lubrifiant d'écoulement et un diluant pour compression directe tels que définis ci-avant et dans les proportions indiquées.

A titre d'exemple, le mélange lyophilisat de MDSE et excipient d'écoulement est composé de :
- une silice colloïdale hydrophobe entre 0,1 et 1%, de préférence 0,5%, en poids par rapport au poids du mélange total ;
- une silice colloïdale hydrophile entre 0,1 et 1%, de préférence 0,5%, en poids par rapport au poids du mélange total et du phosphate dicalcique entre 10 et 90%, de préférence entre 15 et 75%, de préférence 25 à 50%, en poids par rapport au poids du mélange total ;
- le talc entre 0,1 et 1%, de préférence 0,5%, en poids par rapport au poids du mélange total et des maltodextrines nébulisées entre 10 et 90%, de préférence entre 15 et 75%, en poids par rapport au poids du mélange total.

Dans ce contexte, le développement de gélules gastro-résistantes préparées à partir d'une composition lyophilisée constitue un progrès certain en termes de caractéristiques organoleptiques de la préparation, de disponibilité du matériel à transplanter, de conservation de la préparation et de confort de l'individu à traiter.

La lyophilisation permet d'éliminer l'eau présente dans les selles. En effet, les selles normales contiennent entre 75 et 85% d'eau. Après lyophilisation, 50g de selles correspondent environ à 7,5-12,5g de microbiote lyophilisé auxquels s'ajoutent les cryoprotecteurs.

Comparativement à l'administration de selles selon les modalités classiques, qu'il s'agisse de selles fraîches ou de selles congelées, l'administration sous forme d'un lyophilisat pouvant être inclus dans des gélules gastro-résistantes facilite donc la standardisation de la préparation et son acceptabilité par l'individu à traiter. Ainsi, la morbidité devrait être diminuée et le coût de réalisation de la transplantation fécale réduit quand l'administration orale sous forme d'un lyophilisat sera bien établie dans la pratique médicale. En effet l'administration *per os* de la TMF sous forme d'une préparation lyophilisée ne sera plus associée à des gestes techniques requérant un personnel spécialisé et nécessitant une hospitalisation de jour, comme c'est le cas actuellement pour l'administration par voie naso-duodénale, coloscopique ou rectale de préparations issues de selles fraîches ou congelées. Comparativement à l'administration de gélules congelées, l'administration sous forme d'un lyophilisat facilitera le processus de fabrication et de stockage, et donc la standardisation de la préparation. En outre, l'utilisation de préparations lyophilisées permettra une accessibilité améliorée du matériel nécessaire aux TMF, point crucial pour répondre aux situations d'urgence pouvant être rencontrées, par exemple, dans le cadre de récidives d'infection à *Clostridium difficile.*

Ainsi, la présente description a également pour objet une méthode de conservation de selles par lyophilisation comprenant l'utilisation d'un mélange de cryoprotecteurs tel que défini précédemment. Cette méthode permet de conserver les selles lyophilisées au minimum pendant 3 mois et pour une période pouvant aller jusqu'à 7 mois, voire au-delà en assurant le maintien de la viabilité et de la diversité du microbiote comme décrit précédemment.

Les selles conservées lyophilisées selon la description peuvent être administrées dans le cadre d'une TMF allogénique ou autologue. En effet, la TMF peut consister en un transfert de selles entre deux individus de la même espèce ou bien en une administration à un même individu initialement donneur sain à un temps donné lorsqu'il devient un individu à traiter à un temps ultérieur. Ainsi, un individu en bonne santé pourra disposer ultérieurement de la composition lyophilisée comprenant une préparation de selles issue de son don, au moment où il en aura besoin.

La présente description a pour autre objet une composition lyophilisée ou une gélule gastro-résistante telle que définie précédemment pour son utilisation comme médicament.

La présente description se rapporte ainsi à la composition lyophilisée ou à la gélule gastro-résistante précédemment décrite pour son utilisation dans le traitement d'une maladie ou d'un trouble lié à un déséquilibre du microbiote intestinal.

En effet, le microbiote intestinal constitue un écosystème en équilibre et qui s'autorégule en permanence. Cet équilibre est sous la menace d'agressions pouvant conduire à sa rupture. La dysbiose se définit comme une perte de diversité ou un déséquilibre du microbiote qui peut résulter de l'excès de micro-organismes délétères et/ou de l'insuffisance relative de micro-organismes bénéfiques à l'hôte. Ainsi, les indications, pour l'utilisation de la composition lyophilisée selon la description sont celles qui sont associées à une dysbiose. A ce titre, on peut citer en tant que « maladie ou trouble lié à un déséquilibre du microbiote intestinal », la colite à *Clostridium difficile* et ses récidives, les maladies inflammatoires chroniques de l'intestin telles que la maladie de Crohn et la rectocolite hémorragique, le syndrome de l'intestin irritable, le portage de bactéries multirésistantes, la constipation, la diarrhée du voyageur, les infections digestives, les maladies auto-immunes, la diverticulose, le diabète de type 1 et de type 2, le syndrome métabolique, l'obésité, la polyarthrite rhumatoïde, la spondylarthrite ankylosante et les sacroiléites, le syndrome de fatigue chronique, l'halitose, l'allergie, l'acné, les insomnies, les dépressions et des maladies neuropsychiatriques telles que par exemple, la maladie de Parkinson et les maladies du spectre de l'autisme.

Dans un mode de réalisation, l'invention se rapporte à une composition lyophilisée ou à une gélule gastro-résistante précédemment décrite pour son utilisation dans le traitement d'une maladie ou d'un trouble lié à un déséquilibre du microbiote intestinal choisi parmi la colite à *Clostridium difficile* et ses récidives, les maladies inflammatoires chroniques de l'intestin telles que la maladie de Crohn et la rectocolite hémorragique, le syndrome de l'intestin irritable, le portage de bactéries multirésistantes, la constipation, la diarrhée du voyageur, les infections digestives, les maladies auto-immunes, la diverticulose, le diabète de type 1 et de type 2, le syndrome métabolique, l'obésité, la polyarthrite rhumatoïde, la spondylarthrite ankylosante et les sacroiléites, le syndrome de fatigue chronique, l'halitose, l'allergie, l'acné, les insomnies, les dépressions et les maladies neuropsychiatriques telles que la maladie de Parkinson et les maladies du spectre de l'autisme.

De manière préférée, la composition lyophilisée de la description est utilisée dans le traitement de la colite à *Clostridium difficile.* Dans ce contexte, la présence de métabolites bactériens préservés dans les lyophilisats peut avoir un effet bénéfique majeur pour lutter contre C. *difficile.*

La composition lyophilisée peut aussi être utilisée dans le traitement des maladies inflammatoires chroniques intestinales, du portage des bactéries multirésistantes, du diabète de type 1 et de type 2, du syndrome métabolique, de l'obésité et des maladies du spectre de l'autisme.

La préservation de l'environnement associé au microbiote peut avoir un effet contributif majeur dans l'effet thérapeutique de la TMF.

La composition lyophilisée ou la gélule gastro-résistante selon la description peut en outre être utilisée en relai d'un traitement antibiotique oral, tel que le métronidazole ou la vancomycine (traitement de première intention dans les infections à *Clostridium difficile*) en particulier pour le traitement des récidives de colite à *Clostridium difficile,* où les individus sont devenus insensibles à un traitement répété par antibiotiques.

Dans un mode de réalisation, l'invention se rapporte à une composition lyophilisée ou la gélule gastro-résistante pour son utilisation telle que décrite précédemment, en relai d'un traitement antibiotique oral, en cas de récidives d'infections à *Clostridium difficile.*

Une composition de MDSE lyophilisée selon la description est destinée à être administrée à un humain ou un animal.

Dans un contexte où les plans d'action sur l'antibiothérapie visant à limiter le risque de développement des bactéries multi-résistantes se développent, l'utilisation d'une telle composition lyophilisée constitue une alternative de choix en médecine vétérinaire, en particulier dans les élevages biologiques. Ainsi, la composition lyophilisée selon la description peut être utilisée chez l'humain, mais aussi chez les animaux tels que les porcins, bovins, ovins, rongeurs, lagomorphes, carnivores, équidés, oiseaux, les reptiles plus particulièrement chez le porc, le bœuf, le poulet, le rat, le lapin, le chien, le chat, le cheval. La TMF étant une méthode utilisée de manière empirique depuis longtemps et en développement en médecine vétérinaire, l'administration de selles sous forme lyophilisée est à l'évidence de nature à faciliter sa mise en œuvre.

Pour faciliter une administration chez l'animal, une composition lyophilisée de MDSE peut être préparée sous la forme de granulés ne nécessitant pas de réhydratation ou de spray. L'homme du métier saura adapter la forme galénique de sorte à la rendre la plus appropriée possible en fonction de l'animal à traiter.

Un autre objet de la présente description concerne un procédé de préparation d'une composition de MDSE lyophilisée selon la description, comprenant les étapes suivantes :
- recueil du MDSE, en particulier de selles ;
- dilution du MDSE avec un mélange de cryoprotecteurs tel que défini précédemment dans un rapport pondéral M/T compris entre 35/65 et 45/55, et de manière préférée dans un rapport pondéral M/T de 40/60 ;
- lyophilisation du mélange obtenu à l'étape précédente et obtention de la composition lyophilisée.

Selon un mode de réalisation particulier, la lyophilisation peut être réalisée en appliquant une température de congélation allant de -20°C à -80°C, pendant une durée allant de plusieurs dizaines de minutes à plusieurs heures. Dans un mode de réalisation préférée, l'étape de congélation est réalisée à -80°C pendant 30 min. L'homme du métier saura adapter ces conditions.

Comme indiqué précédemment, le mélange de MDSE et de cryoprotecteurs est tel qu'il comprend 15 à 35% en poids de MDSE et 85 à 65% en poids de cryoprotecteurs par rapport au poids total de la composition lyophilisée, de préférence une teneur pondérale de 18 à 28% et de 82 à 72%. Ces teneurs pondérales peuvent varier en fonction de différents paramètres, notamment le degré d'hydratation de l'échantillon de MDSE ainsi que sa teneur en lipides, en fibres et en débris cellulaires qui peut varier, notamment dans le cas des selles. L'homme du métier saura adapter ces teneurs pondérales afin d'optimiser la composition à lyophiliser.

Dans un mode de réalisation particulier, la présente description a trait à un procédé de préparation d'une gélule gastro-résistante comprenant une composition lyophilisée selon la description, comprenant les étapes suivantes :
- préparation d'une composition lyophilisée ;
- pulvérisation du lyophilisat;
- éventuellement ajout d'un ou de plusieurs excipients facilitant l'écoulement du lyophilisat;
- remplissage de l'enveloppe de la gélule gastro-résistante avec ledit mélange lyophilisé ;
- fermeture des gélules.

Dans un mode de réalisation, l'invention concerne un procédé de préparation d'une gélule gastro-résistante telle que décrite précédemment, comprenant les étapes suivantes :
a. recueil d'un microbiote dans son écosystème ;
b. dilution dudit microbiote dans son écosystème avec un mélange de cryoprotecteurs comprenant de la maltodextrine et du tréhalose dans un rapport pondéral M/T compris entre 35/65 et 45/65 ;
c. lyophilisation du mélange obtenu à l'étape b ;
d. pulvérisation du lyophilisat obtenu à l'étape c;
e. Eventuellement ajout d'un ou de plusieurs excipients facilitant l'écoulement du lyophilisat;
f. remplissage de l'enveloppe de la gélule gastro-résistante avec ledit mélange lyophilisé ;
g. fermeture des gélules,
le microbiote dans son écosystème étant le microbiote intestinal, le microbiote fécal ou le microbiote vaginal.

Dans un mode de réalisation, l'invention concerne un procédé tel que décrit précédemment dans lequel ledit microbiote dans son écosystème consiste en des selles, et le mélange de cryoprotecteurs consiste en un mélange de maltodextrine (M) et de tréhalose (T) dans un rapport pondéral M/T de 40/60.

Des modes de réalisation particuliers sont décrits dans les exemples qui suivent. Ils ont pour seul objectif d'illustrer la description et de permettre sa compréhension et ne doivent en aucun cas être considérés comme limitatifs.

### DESCRIPTION DES FIGURES

**Figure 1**: Représentation des principaux groupes bactériens et levures retrouvés après culture dans les selles natives et après dilution en NaCl 9‰ ou dans le mélange de cryoprotecteurs de maltodextrine à 6,7% et de tréhalose à 10% (MT). Les résultats présentés à la figure 1 correspondent à ceux obtenus pour la selle 3.
**Figure 2**: Représentation par rapport à la selle native de la charge bactérienne viable après reconstitution du lyophilisat en fonction de la nature du cryoprotecteur : maltodextrine 5% (M), tréhalose à 5% (T5), tréhalose à 10% (T10), mélange de maltodextrine à 6,7% et de tréhalose à 10% (MT) ou lait (L).
**Figure 3**: Représentation de l'évolution de la charge bactérienne au cours du temps en fonction de la nature du cryoprotecteurs : tréhalose à 5% (T5), tréhalose à 10% (T10) et mélange de maltodextrine 6,7% et de tréhalose à 10% (MT). J0 représente la pré-lyophilisation ; M0, la post-lyophilisation ; M4, 4 mois post-lyophilisation et M7, 7 mois post-lyophilisation.
**Figure 4****:** Représentation du pourcentage d'EOS à M0 en fonction de la nature du cryoprotecteur : maltodextrine à 5% (M), tréhalose à 5% (T5), tréhalose à 10% (T10), mélange maltodextrine à 6,7% et de tréhalose à 10% (MT). J0 représente la pré-lyophilisation ; M0, la post-lyophilisation.
**Figure 5****:** Représentation des principaux groupes bactériens retrouvés dans la selle native et dans les lyophilisats immédiatement après préparation (M0) en fonction de la nature du cryoprotecteur utilisé : tréhalose à 5% (T5), tréhalose à 10% (T10) et mélange de maltodextrine à 6,7% et de tréhalose à 10% (MT). Les résultats présentés à la figure 5 correspondent à ceux obtenus pour la selle 3.
**Figure 6****:** Représentation des principaux groupes bactériens retrouvés dans la selle native et dans les lyophilisats après préparation en fonction de la nature du cryoprotecteur utilisé : tréhalose à 5% (T5), tréhalose à 10% (T10) et mélange de maltodextrine à 6,7% et de tréhalose à 10% (MT). Les résultats sont présentés immédiatement après lyophilisation et après stockage à 4°C pendant 3 mois (+3) et 8 mois (+8). Les résultats présentés à la figure 6 correspondent à ceux obtenus pour la selle 3.

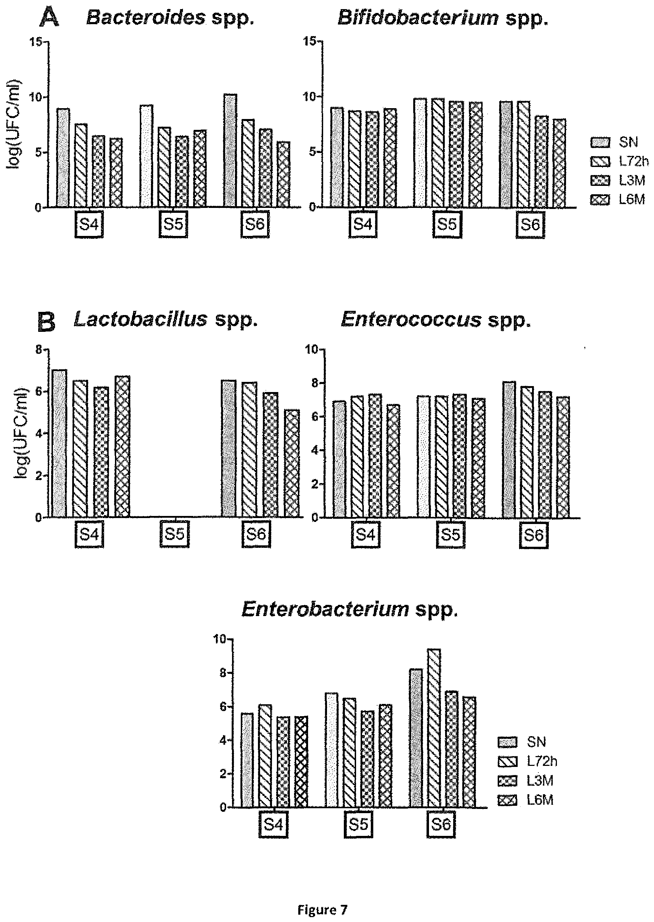

**Figure 7****:** Représentation de l'impact de la lyophilisation (L72h) puis de la conservation des lyophilisats pendant 3 mois (L3M) et 6 mois (L6M) sur la viabilité des principaux groupes bactériens de trois prélèvements fécaux (S4, S5, 56) par rapport à la selle native (SN). (A) Effet sur différentes populations bactériennes anaérobies strictes. (B) Effet sur différentes populations bactériennes aéro-anaérobies facultatives.
**Figure 8****:** Représentation de l'impact de la lyophilisation (L72h) puis de la conservation des lyophilisats pendant 3 mois (L3M) et 6 mois (L6M) sur la viabilité des bactéries extrêmement sensibles à l'oxygène (EOS) des différents prélèvements fécaux (S4, S5, S6) par rapport à la selle native (SN).
**Figure 9****:** Evaluation de la production de métabolites bactériens par chromatographie en phase gazeuse de selles natives (SN) et lyophilisées (L72h).
**Figure 10****:** Evaluation de l'effet *anti-Clostridium difficile* des selles natives (S ou SN) et lyophilisées (L72h)

### EXEMPLE 1

### 1. Matériels et Méthodes

### 1.1. Prélèvements fécaux

La sélection des conditions de lyophilisation a été réalisée à partir de l'étude de 3 selles provenant de volontaires sains indépendants et placées immédiatement après émission en présence d'un système GENbagAnaer® mis dans le pot de prélèvement pour incubation. La transmission au laboratoire a été réalisée immédiatement après émission, dans un délai inférieur à 1 heure. Une fraction de chaque selle est conservée sous GENbagAnaer® comme témoin pour analyse immédiate.

### 1.2. Choix des cryoprotecteurs

Les cryoprotecteurs utilisés pour cette étude sont les suivants :
- le glycérol à 10% (G).
- le lait écrémé et pasteurisé en poudre reconstitué à 10% (L).
- la maltodextrine, à la concentration de 5% (M).
- le tréhalose, aux concentrations de 5% (T5) et 10% (T10).
- le mélange constitué de maltodextrine à une concentration de 6,7% et de tréhalose à une concentration de 10%, soit dans un rapport pondéral M/T de 40/60 (MT).
- une solution de NaCl à une concentration de 9‰, utilisée en tant que contrôle car habituellement employée pour diluer le matériel fécal frais.

Chaque solution a été conditionnée en pots stériles et conservée à +4°C jusqu'à utilisation.

### 1.3. Préparation du lyophilisat fécal

Les selles ont été diluées 10 fois dans la solution de cryoprotection et homogénéisées à l'aide d'un homogénéiseur de type Ultraturax® équipé d'une tige à usage unique. Les suspensions ont été déposées dans des flacons à lyophilisation sous un volume de 3 ml correspondant à 300 mg de selle native.

Pour chaque condition testée, et pour chacune des 3 selles analysées, un flacon de suspension a été immédiatement placé en présence d'un système GENbagAnaer® afin d'évaluer l'effet des différents cryoprotecteurs sur la viabilité des bactéries du microbiote de chaque selle diluée et non lyophilisée.

Les flacons restants sont placés immédiatement à -80°C. Après 30 minutes de congélation, les flacons sont placés dans le lyophilisateur (FreeZone® 6 Liter, Freeze Dry System, Model 77530, Labconco Corporation, Kansas City, MO, USA) dont le piège est déjà refroidi pour une durée de 24h sous cycle automatique. A l'issue de cette période de lyophilisation, les flacons sont bouchés sous vide puis sertis à l'aide de capsules en aluminium.

Pour chaque selle et chaque condition de cryoconservation, une série de flacons est transmise au laboratoire de microbiologie pour analyse du microbiote et 2 séries sont conservées à 4°C pour des études de stabilité à 3-4 mois et 7-8 mois.

### 1.4. Etudes in vitro de la viabilité du microbiote en fonction des conditions de lyophilisation

La première étape de l'étude microbiologique a porté sur l'analyse des selles diluées non lyophilisées pour tester l'effet de la dilution en présence des différents cryoprotecteurs sur le maintien de la diversité du microbiote et de sa viabilité. Pour cela, une aliquote des dilutions réalisées en milieu lait à 10% (L), glycérol 10% (G), maltodextrine 5% (M), tréhalose 5% (T), tréhalose 10% (T10) et mélange de maltodextrine 6,7% et de tréhalose 10%, soit un rapport pondéral M/T de 40/60 (MT) a été placée immédiatement après la préparation du lyophilisat fécal sous anaérobiose et les cultures microbiennes ont été réalisées immédiatement.

La deuxième étape du travail a porté sur l'étude des lyophilisats. Les analyses ont été réalisées dans la semaine suivant la préparation puis après 3-4 mois et 7-8 mois de stockage à 4°C. Chaque lyophilisat est reconstitué avec 3 ml d'eau stérile peptonée, ce qui correspond au volume de selle diluée présent initialement dans chaque flacon.

Pour l'analyse du microbiote aérobie et anaérobie strict, les selles non lyophilisées et les lyophilisats reconstitués ont été dilués de 10 en 10 et ensemencés sur différents milieux sélectifs et non sélectifs (Rougé et *al.,* 2010). Les milieux incubés en aérobie et en cabinet anaérobie (N₂/CO₂/H₂ :80/10/10) à 37°C sont indiqués dans le tableau 1.

**Tableau 1 : Milieux utilisés pour l'analyse du microbiote par culture**

| Eléments du microbiote recherchés | Base gélosée | Incubation/lecture |
|---|---|---|
| Anaérobies totales | Colombia + cystéine (Cc) + sang + néomycine (100 mg/ml) | |
| *Bacteroides* sp., *Prevotella* sp. | Cc + sang + kanamycine (100 mg/ml) + vancomycine (7 mg/ml) | 37°C Cabinet anaérobie |
| *Clostridium difficile* | Cc + sang + mélange Clostridium difficile (CLO-M Biomérieux) | Lecture et dénombrement à 48h et 5 jours |
| Autres *Clostridium* | CC + mupirocine (100 mg/ml) + lait entier (5ml) + rouge neutre (40 mg/ml) | |
| *Bifidobacterium* sp. | WCB (Wilkins-Chalgren for Bifidobacteria) + mupirocine (100 mg/ml) | |
| *Lactobacillus* sp. | MRS (Gélose de Man, Rosaga, Sharpe) | |
| Aérobies totales | Trypcase soja | 37°C Aérobiose |
| Entérobactéries | Drigalski | |
| *Enterococcus* sp. | Enterococcosel | |
| *Staphylococcus* sp. | Chapman 110 | Lecture à 24h |
| Levures | Sabouraud Chloramphenicol | |

La quantification des bactéries extrêmement sensibles à l'oxygène (EOS) telles que les bactéries du groupe *Clostridium leptum,* a été réalisée par culture différentielle en présence ou absence d'oxygène. Pour cela, les échantillons de la selle fraîche (T0) et les différents échantillons lyophilisés réhydratés dans l'eau peptonée ont été successivement dilués dans un milieu de culture très riche non sélectif (YBHI correspondant à un milieu cœur-cervelle enrichi en extrait de levure) complété en cellobiose, maltose et cystéine. Ce milieu a été mis au point pour permettre la culture de bactéries extrêmement difficiles à cultiver de type *Faecalibacterium prausnitzii.* Une fois des dilutions *ad hoc* effectuées en anaérobiose stricte, leur capacité à résister ou non à la présence d'oxygène a été évaluée par culture sur 2 boites en parallèle, une étant soumise à une atmosphère aérobie pendant 60 minutes et l'autre non. Cette méthode, permet d'évaluer le pourcentage de bactéries viables anaérobies strictes au sein des différents échantillons, à différents temps après la lyophilisation.

### 2. Résultats

### 2.1. Effet des différents cryoprotecteurs sur la diversité et la viabilité du microbiote avant lyophilisation.

Une dilution de la selle dans les différents cryoprotecteurs évalués, et notamment le mélange d'intérêt MT, n'a pas eu d'impact significatif sur la charge bactérienne, ni sur l'équilibre des principaux genres cultivables du microbiote, comparativement à la selle native ou à la selle diluée en NaCl à 9‰ (correspondant à la préparation utilisée pour la TMF avec selles fraîches) (Figure 1). Les groupes majoritaires du microbiote restent viables.

### 2.2. Effet de la lyophilisation sur la viabilité du microbiote en fonction des cryoprotecteurs utilisés

La charge bactérienne totale a été évaluée dans la semaine suivant la lyophilisation, soit à M0. Les résultats sont présentés sur la Figure 2. Les trois cryoprotecteurs testés, à savoir une solution de tréhalose à 5% (T5), une solution de tréhalose à 10% (T10) et un mélange de maltodextrine 6,7% et de tréhalose 10% (TM), permettent d'assurer une stabilité supérieure à 90% de la charge bactérienne totale des espèces cultivables lors de la lyophilisation ; ce résultat correspond à une perte d'environ 1 log par rapport à la charge existante dans une selle native ou une selle diluée en NaCl à 9‰ avant lyophilisation.

Cette charge bactérienne totale, mesurée jusqu'à 7 mois post-lyophilisation a peu évolué (Figure 3).

De plus, ces 3 cryoprotecteurs permettent une préservation efficace des bactéries EOS lors de la lyophilisation (Figure 4).

### 2.3. Effet de la lyophilisation sur la diversité du microbiote en fonction des différents cryoprotecteurs utilisés

Parmi les 3 cryoprotecteurs testés ci-dessus, le mélange cryoprotecteur de maltodextrine à 6,7% et de tréhalose à 10%, (soit dans un rapport pondéral de 40/60) est celui qui donne les meilleurs résultats quant au maintien en vie des principaux genres constitutifs du microbiote fécal, malgré une diminution de certains genres anaérobies stricts d'environ 1 à 2 log, si on considère la selle 3 (Figure 5) (et jusqu'à 4 log pour les autres selles - résultats non présentés). A l'inverse, le glycérol à 10% se révèle peu protecteur, que ce soit sur les genres aérobies ou les genres anaérobies (résultat non montré).

Les genres anaérobies stricts (*Ciostridium* sp. et *Bacteroides* sp.) restent dominants lorsque l'étude est réalisée dans la semaine suivant la lyophilisation (Figure 6). La conservation à 4°C du lyophilisat n'a que peu de répercussion avec une perte supplémentaire de l'ordre de 1,5 log pour les *Bacteroides* sp. après 3 mois de conservation à 4°C et une perte d'1 log à 8 mois pour les *Clostridium* sp (Figure 6). Dans tous les cas, les genres dominants du microbiote restent en état de dominance au cours de la conservation.

Les résultats obtenus avec la selle 2, riche en *Bifidobacterium* sp. ont montré la stabilité de ce groupe, jusqu'à 6 mois post-lIyophilisation dans le mélange MT (résultat non montré).

Pris dans leur ensemble, ces résultats démontrent que le mélange MT est celui qui permet la meilleure préservation du microbiote fécal lyophilisé, aussi bien en termes de viabilité que de diversité et ce pour une durée pouvant aller jusqu'à 6 mois après lyophilisation.

### EXEMPLE 2

### 1. Matériels et Méthodes

En application de la méthode décrite dans l'exemple 1, trois prélèvements fécaux supplémentaires identifiés comme S4, S5 et S6 ont été dilués en présence du mélange cryoprotecteur de maltodextrine à 6,7% et de tréhalose à 10% (soit dans un rapport pondéral M/T de 40/60), puis lyophilisés.

### 2. Résultats

### 2.1. Stabilité de l'écosystème bactérien dans les lyophilisats après conservation pendant 6 mois

### 2.1.1. Analyse de la viabilité et de la diversité bactérienne dans des lyophilisats après conservation pendant 6 mois

La population anaérobie stricte a été analysée sur la base du dénombrement des groupes bactériens représentatifs : *Bifidobacterium* spp. et *Bacteroides* spp..

Concernant le genre *Bifidobacterium* spp., il reste partie intégrante du microbiote dominant et l'absence d'évolution significative sur la période de conservation de 6 mois est confirmée.

Pour le genre *Bacteroides* spp., il existe des variations inter-individuelles aboutissant à une perte d'environ 1,5 à 4 log à 6 mois.

Les populations aéro-anaérobies facultatives des différents échantillons ont été analysées sur la base du dénombrement des groupes bactériens *Lactobacillus* spp., *Enterococcus* spp. et *Enterobacteriaceae.* La stabilité et la viabilité de ces genres et groupes bactériens post-lyophilisation tout au long de la période de conservation sur 6 mois sont confirmées.

Ces résultats montrent que le processus de lyophilisation permet de conserver en grande majorité la viabilité des différentes populations bactériennes ciblées sans induction de modifications dans les grands équilibres entre les principaux groupes bactériens et que cette conservation reste stable sur une période de conservation de 6 mois (Figure 7).

### 2.1.2. Etude des bactéries extrêmement sensibles à l'oxygène

Une des spécificités du microbiote fécal est la présence de bactéries extrêmement sensibles à l'oxygène (EOS) pouvant être affectées par le processus de lyophilisation.

Ces bactéries EOS sont détectées à la fois pour la selle native et dans les lyophilisats fécaux pour tous les échantillons analysés (Figure 8). Quand ces bactéries EOS sont exprimées en pourcentage par rapport à la quantité totale de bactéries anaérobies (UFC/ml), leur niveau reste stable tout au long de la conservation (3 à 6 mois selon les échantillons) avec une quantité de bactéries anaérobies strictes qui elle-même n'est pas affectée par le processus de conservation.

Ces résultats montrent que la lyophilisation n'affecte pas les populations bactériennes très sensibles à l'oxygène.

### 2.2. Stabilité des fonctions de l'écosystème bactérien dans les lyophilisats

### 2.2.1. Détection de métabolites dans les lyophilisats

La production de métabolites bactériens, à savoir des acides gras à chaînes courtes, a été évaluée dans le lyophilisat de 72h comparativement à la selle native. Sur les 3 prélèvements analysés, la lyophilisation induit une conservation des produits de fermentation avec une concentration des acides gras à chaînes courtes augmentée d'un facteur 2 à 4 pour l'acétate et le propionate selon les selles et d'un facteur 2 à 6 pour le butyrate. Les métabolites bactériens, bien que volatils sont présents dans les lyophilisats. L'augmentation de leur concentration serait une conséquence de la lyophilisation qui aboutit à une concentration des métabolites (Figure 9).

### 2.2.2. Détection de l'effet anti-Clostridium difficile dans les lyophilisats

L'effet *anti-Clostridium difficile* des selles natives ainsi que des lyophilisats a été analysé *in vitro* en présence de 2 souches de *Clostridium difficile,* une souche toxinogène (souche 630) et l'autre non toxinogène (souche PCD1). Quand les souches sont inhibées dans leur croissance, un cercle pâle apparait autour de la zone d'inoculation : il s'agit du cercle d'inhibition.

Différents contrôles, connus pour leurs effets *anti-Clostridium* ont été utilisés systématiquement comme contrôles positifs. L'acide acétique (AA) ainsi que l'acide lactique (AL) ont ainsi été utilisés à différentes concentrations (300, 400, 500 mM). Sur chaque boite, un cercle d'inhibition a été observé en présence d'acide acétique et/ou d'acide lactique.

Des souches pures ont été utilisées comme contrôle, à savoir, une souche de Lactobacille et une souche de Bifidobactérie (CB) qui n'entrainent pas d'apparition de cercles d'inhibition.

En présence de selles natives (S ou SN) comme en présence des selles ayant subi une congélation à -80°C pendant 72h (T1, T2 et T3), aucun cercle d'inhibition n'a été observé alors qu'un cercle d'inhibition est visible en présence des lyophilisats (Figure 10).

Les résultats des différentes expériences ont montré la présence d'une zone d'inhibition pour l'acide acétique et l'acide lactique, ainsi que pour les lyophilisats de l'ensemble des selles analysées. La capacité d'inhibition du développement *in vitro* de *C. difficile* par une selle est donc potentialisée par la lyophilisation.

L'ensemble des résultats présentés dans l'exemple 2 confirme la stabilité et la viabilité du microbiote au sein du lyophilisat sur une période d'au moins 6 mois et montre le maintien des capacités fonctionnelles des bactéries au sein du lyophilisat avec notamment une préservation de métabolites bactériens d'intérêt et un effet anti*-Clostridium difficile* dans un modèle d'étude *in vitro.*

### EXEMPLE 3

### 1. Matériels et Méthodes

### 1.1. Lyophilisats de selles

Quatre selles issues de donneurs sains ont été traitées en application de la méthode décrite dans l'exemple 1, afin d'obtenir quatre lyophilisats fécaux : S4, S5, S6 et S7, S7 représentant un prélèvement fécal supplémentaire par rapport à l'exemple 2.

### 1.2. Choix des excipients

### Diluants

Le choix des diluants a été basé sur leur caractère inerte et en particulier sur leur absence d'effets propres au niveau intestinal.

Les diluants sélectionnés sont un excipient minéral, le phosphate dicalcique (Encompress® anhydre, JRS Pharma, Rosenberg, Allemagne), et des maltodextrines nébulisées (Emdex®, JRS Pharma, Rosenberg, Allemagne), ce dernier type d'excipient entrant déjà dans la composition du lyophilisat, La masse volumique et le temps d'écoulement de ces deux excipients purs sont respectivement, 0,79 g/mL et 3 secondes 18/100 g pour Encompress® anhydre, et 0,68 g/mL et 6 secondes 03/100 g pour Emdex®.

### Lubrifiants

Trois lubrifiants d'écoulement ont été sélectionnés : le talc (Cooper, France), une silice colloïdale hydrophile (Aérosil® 200 Pharma, Kraemer & Martin GmbH, St-Augustin, Allemagne), et une silice colloïdale hydrophobe (Aérosil® R972, Degussa-Hüls, Courbevoie, France).

### 1.3. Mesure des temps d'écoulement et mélange des lyophilisats avec les excipients

Cet essai a été effectué selon la monographie 2.9.16 de la Pharmacopée européenne, sur les lyophilisats seuls, puis après addition d'un lubrifiant d'écoulement utilisé à 0,5%, et si besoin d'un diluant testé dans les rapports pondéraux 75/25, 50/50 et 25/75. Les mélanges ont été réalisés manuellement.

La masse volumique des mélanges finaux présentant une bonne fluidité a été mesurée sur la totalité des mélanges, puis ensuite rapportée par calcul à 1 mL.

### 1.4. Mise en gélules des mélanges présentant un bon écoulement

Des gélules N°00, associant une contenance importante de 0,95 mL et une taille compatible avec une ingestion relativement facile (23,3 mm de longueur) ont été choisies. Elles ont été remplies manuellement sur gélulier avec les mélanges présentant un bon écoulement.

### 2. Résultats

### 2.1. Etude des caractéristiques d'écoulement et de masse volumique des lyophilisats

Le tableau 1 regroupe les résultats des essais réalisés sur les lyophilisats.

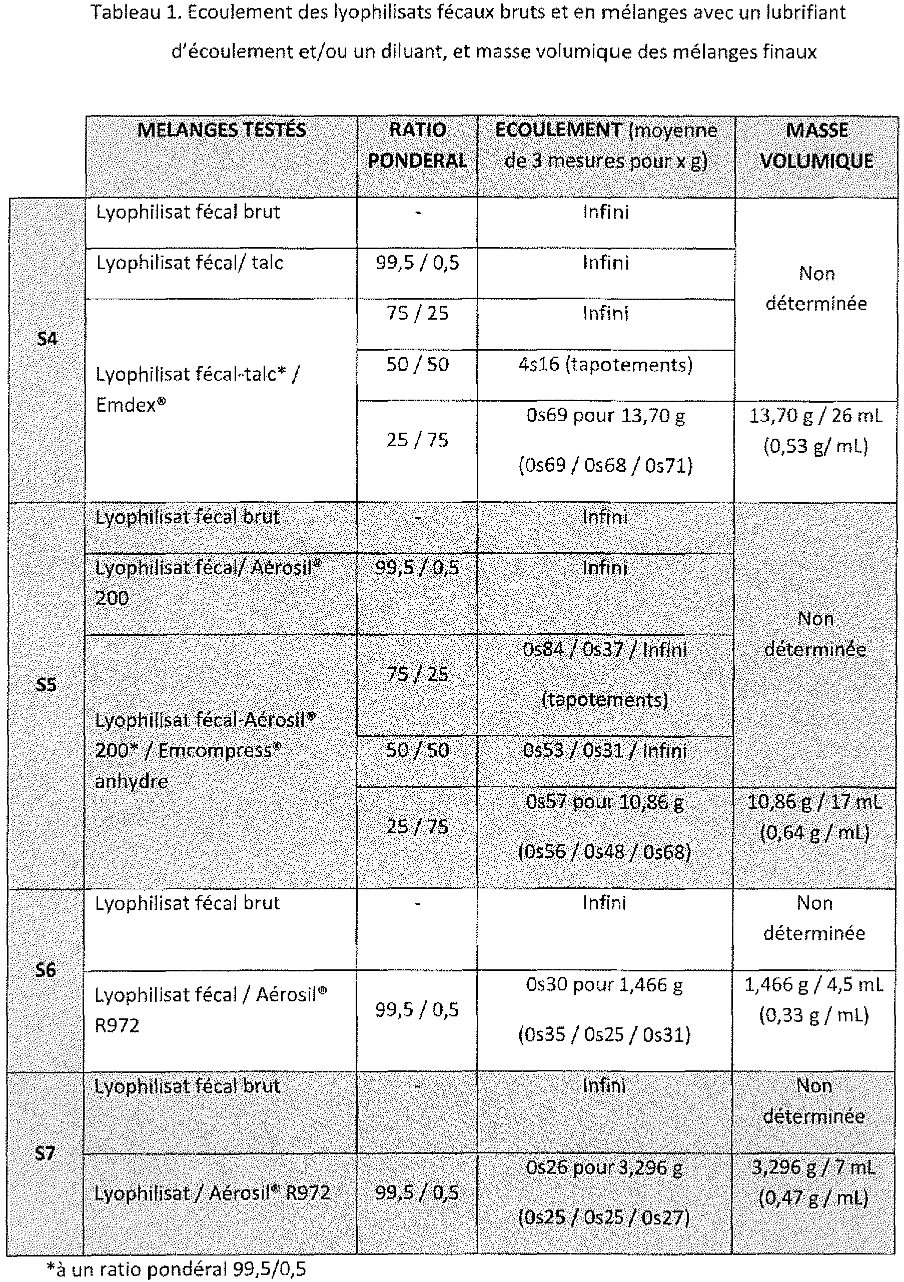

Aucun des lyophilisats fécaux bruts testé seul ne présente de propriété d'écoulement. Il est donc impossible de les mettre en gélules en l'état.

En dépit de leur rôle de lubrifiant d'écoulement, le talc et l'Aérosil® 200 utilisés à 0,5% n'ont pas permis d'améliorer la fluidité des lyophilisats (S4 et S5).

Les diluants Emdex® et Encompress® anhydre sont efficaces pour améliorer l'écoulement. L'amélioration se produit dès le ratio 50/50 pour le mélange lyophilisat S4-talc / Emdex®, et dès le ratio 75/25 pour le mélange lyophilisat S5-Aérosil® 200 / Erncompress® anhydre. Au ratio 25/75 entre lyophilisat et diluant, le mélange devient fluide avec un temps d'écoulement de 0s69 pour 13,70 g (S4) et 0s57 pour 10,86 g (S5).

Enfin, le lubrifiant d'écoulement Aérosil® R972, utilisé seul à 0,5 %, a permis une amélioration très nette de la fluidité des lyophilisats S6 (0s30 pour 1,466 g) et S7 (0s26 pour 3,296 g).

La masse volumique des mélanges varie de 0,53 à 0,64 g/ mL pour les mélanges « lyophilisat/lubrifiant d'écoulement/dituant », et de 0,33 à 0,47 g/ mL pour les mélanges « lyophilisat/lubrifiant d'écoulement ».

### 2.2. Mise en gélule des mélanges

Le tableau 2 présente le nombre de gélules préparées pour chaque échantillon et la masse de lyophilisat incorporée par gélule. La masse théorique de matériel frais mise en œuvre initialement est reportée en dernière colonne.

**Tableau 2. Caractéristiques des gélules préparées à partir des échantillons lyophilisés**

| | **NOMBRE DE GELULES PREPAREES** | **MASSE DE MELANGE / GELULE** | **MASSE DE LYOPHILISAT / GELULE** | **MASSE THEORIQUE DE MATERIAL FRAIS DANS n GELULES** |
|---|---|---|---|---|
| **S4-talc / Emdex®** | 20 | 500 mg | 125 mg | 1754 mg |
| **S5-Aérosil® 200 / Emcompress® anhydre** | 18 | 603 mg | 151 mg | 3000 mg |
| **S6-Aérosil® R972** | 5 | 293 mg | 293 mg | 1792 mg |
| **S7-Aérosil® R972** | 7 | 471 mg | 471 mg | 5000 mg |

Le cas des échantillons S6 et S7 est le plus favorable car la dilution du lyophilisat est négligeable du fait de l'ajout de seulement 0,5 % de lubrifiant d'écoulement. Néanmoins, une assez grande variabilité est observée entre les deux échantillons qui, sous un même volume, peuvent représenter 293 mg (S6) ou 471 mg (S7) de matières sèches. Ceci est dû à la différence de masse volumique entre les deux échantillons.

En ce qui concerne les échantillons S4 et S5, la dilution du lyophilisat est plus importante du fait de l'ajout de 75 % de diluant, en plus des 0,5 % de lubrifiant d'écoulement. Sous un même volume, la masse de lyophilisat varie de 125 mg (S4) à 151 mg (S5) de matières sèches, en relation avec la masse volumique de chaque échantillon.

Cette étude montre que les lyophilisats de microbiote fécal réduits en poudre ne présentent pas de propriété de fluidité et ne peuvent donc pas être mis en gélules seuls.

En revanche, il est possible d'améliorer les propriétés d'écoulement des lyophilisats par mélange, soit avec 75% de diluant (maltodextrines Emdex®, phosphate dicalcique anhydre Emcompress® anhydre), soit avec 0,5 % de lubrifiant d'écoulement (silice colloïdale hydrophobe, Aérosil® R972).

### BIBLIOGRAPHIE

Allen-Vercoe E, Petrof EO. Artificial stool transplantation: progress towards a safer, more effective and acceptable alternative. Expert Rev Gastroenterol Hepatol 2013;7:291-3.
ANSM. Rapport. La transplantation de microbiote fécal et son encadrement dans les essais cliniques, Mars 2014, mise à jour juin 2015.
Béal C and Fonseca F. Freezing of probiotic bacteria. P. 179-212. Advances in probiotic technology. Eds Foerst P. and Sativarangkna C, 2015, CRC Press, Boca Raton, USA.
Borody T, Fischer M, Mitchell S, Campbell J. Fecal microbiota transplantation in gastrointestinal disease: 2015 update and the road ahead. Expert Rev Gastroenterol Hepatol. 2015;9:1379-91.
Carvalho A.S., Silva J., Ho P., Texeira P., Malcata F.X. and Gibbs P. Relevant factors for the preparation of freeze-dried lactic acid bacteria. Int. Dairy J 2004, 14: 835-47.
Debast SB, Bauer MP, Kuijper EJ. European Society of Clinical Microbiology and Infectious Diseases: update of the treatment guidance document for Clostridium difficile infection. Clin Microbiol Infect 2014;20Suppl 2:1-26.
Hamilton MJ, Weingarden AR, Sadowsky MJ, KhorutsA. Standardized frozen preparation for transplantation of fecal microbiota for recurrent Clostridium difficile infection. Am J Gastroenterol2012;107:761-7.
Kapel N, Thomas M, Corcos O, Mayeur C, Barbot-Trystram L, Bouhnik Y, Joly F. Practical implementation of faecal transplantation. Clinical Microbiology and Infection2014;20:1098-105.
Kelly CR, Kahn S, Kashyap P, Laine L, Rubin D, Atreja A, Moore T, Wu G. Gastroenterology.2015 Jul;149(1): 223-37.
Le Hir A, Chaumeil JC, Brossard D, Charrueau C, Crauste-Mancier S. Pharmacie galénique, bonnes pratiques de fabrication des médicaments. Elsevier Masson SAS, Issy-les-Moulineaux, France, 10ème édition, 2016.
Lee CH, Steiner T, Petrof EO, Smieja M, Roscoe D, Nematallah A, Weese JS, Collins S, Moayyedi P, Crowther M, Ropeleski MJ, Jayaratne P, Higgins D, Li Y, Rau NV, Kim PT. Frozen vs Fresh Fecal Microbiota Transplantation and Clinical Resolution of Diarrhea in Patients With Recurrent Clostridium difficile Infection: A Randomized Clinical Trial. JAMA. 2016;315:142-149
Ott SJ, Waetzig GH, Rehman A, Moltzau-Anderson J, Bharti R, Grasis JA, Cassidy L, Tholey A, Fickenscher H, Seegert D, Rosenstiel P, Schreiber S. Efficacy of Sterile Fecal Filtrate Transfer for Treating Patients With Clostridium difficile Infection. Gastroenterology. 2016 Nov 17. pii: S0016-5085(16)35354-9. doi: 10.1053/j.gastro.2016.11.010.
Pharmacopée européenne 8ème édition, 2016. Monographie 2.9.16. Ecoulement. EDQM European Directory for the Quality of Medicines and Healthcare, https://www.edqm.eu/fr/Pharmacopee-europeenne-8eme-Edition-1563.html
Rougé C, Goldenberg O, Ferraris L, Berger B, Rochat F, Legrand A, Göbel UB, Vodovar M, Voyer M, Rozé JC, Darmaun D, Piloquet H, Butel MJ, De La Cochetière MF. Investigation of the intetinal microbiota in preterm infants using different methods. Anaerobe 2010;16:362-370.
Sokol H, Galperine T, Kapel N, Bourlioux P, Seksik P, Barbut F, Scanzi J, Chast F, Batista R, Joly F, Joly AC, Collignon A, Guery B, Beaugerie L, pour le GroupeFrançais de Transplantation Fécale (GFTF). Transplantation de microbiote fécal dans le cadre des infections à Clostridium difficile récidivantes : recommandations pour la pratique clinique courante. Hepato Gastro 201 ; 22:278-90.
Surawicz CM, Brandt U, Binion DG, et al. Guidelines for diagnosis, treatment, and prevention of Clostridium difficile infections. Am J Gastroenterol 2013;108:478-98.
Van Nood E, Vrieze A, Nieuwdorp M, Fuentes S, Zoetendal EG, de Vos WM, Visser CE, Kuijper EJ, Bartelsman JF, Tijssen JG, Speelman P, Dijkgraaf MG, Keller J. Duodenal infusion of donor feces for recurrent Clostridium difficile. N Engl J Med 2013;368:407-15.
Vrieze A, de Groot PF, Kootte RS, Knaapen M, van Nood E, Nieuwdorp M. Fecal transplant: a safe and sustainable clinical therapy for restoring intestinal microbial balance in human disease. Best Pract Res Clin Gastroenterol 2013;27:127-37.
Youngster I, Sauk J, Pindar C, Wilson RG, Kaplan JL, Smith MB, Alm EJ, Gevers D, Russell GH, Hohmann EL. Fecal microbiota transplant for relapsing Clostridium difficile infection using a frozen inoculum from unrelated donors: a randomized, open-label, controlled pilot study. Clin Infect Dis 2014a;58:1515-22
Youngster I, Russell GH, Ziv-Baran T, Sauk J, Hohmann EL. Oral, capsulized, frozen fecal microbiota transplantation for relapsing Clostridium difficile infection.JAMA. 2014b;312:1772-8.
Youngster 1, Mahabamunuge J, Systrom HK, Sauk J, Khalili H, Levin J, Kaplan JL, Hohmann EL. Oral, frozen fecal microbiota transplant (FMT) capsules for recurrent Clostridium difficile infection. BMC Med. 2016 Sep 9;14(1):134. doi: 10.1186/s12916-016-0680-9.

## Revendications

1. Utilisation d'un mélange de cryoprotecteurs pour la conservation d'un microbiote dans son écosystème sous forme lyophilisée, **caractérisée en ce que** ledit mélange de cryoprotecteurs comprend de la maltodextrine (M) et du tréhalose (T) dans un rapport pondéral M/T compris entre 35/65 et 45/55, le microbiote dans son écosystème étant le microbiote intestinal, le microbiote fécal ou le microbiote vaginal.

2. Utilisation selon la revendication 1 dans laquelle le microbiote dans son écosystème consiste en des selles.

3. Composition lyophilisée comprenant un microbiote dans son écosystème et un mélange de cryoprotecteurs, **caractérisée en ce que** ledit mélange de cryoprotecteurs comprend de la maltodextrine et du tréhalose, utilisés dans un rapport pondéral M/T compris entre 35/65 et 45/55, le microbiote dans son écosystème étant le microbiote intestinal, le microbiote fécal ou le microbiote vaginal.

4. Composition lyophilisée selon la revendication 3 dans laquelle (i) le microbiote dans son écosystème (MDSE) consiste en des selles, (ii) la quantité en poids de selles représente 15 à 35% de la composition lyophilisée et la quantité en poids du mélange de cryoprotecteurs représente 85 à 65% de la composition lyophilisée et (iii) la maltodextrine (M) et le tréhalose (T) sont utilisés dans un rapport pondéral M/T de 40/60.

5. Gélule gastro-résistante comprenant une composition lyophilisée telle que définie à l'une des revendications 3 ou 4.

6. Gélule gastro-résistante selon la revendication 5, **caractérisée en ce que** ladite composition lyophilisée est mélangée à au moins un excipient d'écoulement.

7. Composition lyophilisée selon l'une des revendications 3 ou 4 ou gélule gastro-résistante selon la revendication 5 ou 6, pour son utilisation dans le traitement d'une maladie ou d'un trouble liés à un déséquilibre du microbiote intestinal choisi parmi la colite à *Clostridium difficile* et ses récidives, les maladies inflammatoires chroniques de l'intestin telles que la maladie de Crohn et la rectocolite hémorragique, le syndrome de l'intestin irritable, le portage de bactéries multirésistantes, la constipation, la diarrhée du voyageur, les infections digestives, les maladies auto-immunes, la diverticulose, le diabète de type 1 et de type 2, le syndrome métabolique, l'obésité, la polyarthrite rhumatoïde, la spondylarthrite ankylosante et les sacroiléites, le syndrome de fatigue chronique, l'halitose, l'allergie, l'acné, les insomnies, les dépressions et les maladies neuropsychiatriques telles que la maladie de Parkinson et les maladies du spectre de l'autisme.

8. Composition lyophilisée ou gélule gastro-résistante pour son utilisation selon la revendication 7, en relai d'un traitement antibiotique oral, en cas de récidives d'infections à *Clostridium difficile.*

9. Procédé de préparation d'une gélule gastro-résistante telle que définie dans la revendication 5 ou 6, comprenant les étapes suivantes :
a. recueil d'un microbiote dans son écosystème ;
b. dilution dudit microbiote dans son écosystème avec un mélange de cryoprotecteurs comprenant de la maltodextrine et du tréhalose dans un rapport pondéral M/T compris entre 35/65 et 45/65 ;
c. lyophilisation du mélange obtenu à l'étape b ;
d. pulvérisation du lyophilisat obtenu à l'étape c;
e. Eventuellement ajout d'un ou de plusieurs excipients facilitant l'écoulement du lyophilisat ;
f. remplissage de l'enveloppe de la gélule gastro-résistante avec ledit mélange lyophilisé ;
g. fermeture des gélules,
le microbiote dans son écosystème étant le microbiote intestinal, le microbiote fécal ou le microbiote vaginal.

10. Procédé selon la revendication 9 dans lequel ledit microbiote dans son écosystème consiste en des selles, et le mélange de cryoprotecteurs consiste en un mélange de maltodextrine (M) et de tréhalose (T) dans un rapport pondéral M/T de 40/60.

## Patentansprüche

1. Verwendung eines Gemisches an Kryoprotektoren zur Konservierung einer Biozönose in ihrem Ökosystem in lyophilisierter Form, **dadurch gekennzeichnet, dass** das Gemisch an Kryoprotektoren Maltodextrin (M) und Trehalose (T) in einem Gewichtsverhältnis M/T zwischen 35/65 und 45/55 umfasst, wobei die Biozönose in ihrem Ökosystem die Darm-Biozönose, die Fäkal-Biozönose oder die vaginale Biozönose ist.

2. Verwendung nach Anspruch 1, wobei die Biozönose in ihrem Ökosystem aus Stühlen besteht.

3. Lyophilisierte Zusammensetzung, umfassend eine Biozönose in ihrem Ökosystem und ein Gemisch an Kryoprotektoren, **dadurch gekennzeichnet, dass** das Gemisch an Kryoprotektoren Maltodextrin und Trehalose umfasst, die in einem Gewichtsverhältnis M/T zwischen 35/65 und 45/55 verwendet werden, wobei die Biozönose in ihrem Ökosystem die Darm-Biozönose, die Fäkal-Biozönose oder die vaginale Biozönose ist.

4. Lyophilisierte Zusammensetzung nach Anspruch 3, wobei (i) die Biozönose in ihrem Ökosystem (MDSE) aus Stühlen besteht, (ii) die Gewichtsmenge an Stühlen 15 bis 35 % der lyophilisierten Zusammensetzung repräsentiert und die Gewichtsmenge des Gemisches an Kryoprotektoren 85 bis 65 % der lyophilisierten Zusammensetzung repräsentiert, und (iii) das Maltodextrin (M) und die Trehalose (T) in einem Gewichtsverhältnis M/T von 40/60 verwendet werden.

5. Gastro-beständige Kapsel, umfassend eine lyophilisierte Zusammensetzung wie in einem der Ansprüche 3 oder 4 definiert.

6. Gastro-beständige Kapsel nach Anspruch 5, **dadurch gekennzeichnet, dass** die lyophilisierte Zusammensetzung mit mindestens einem Flusshilfsstoff gemischt ist.

7. Lyophilisierte Zusammensetzung nach einem der Ansprüche 3 oder 4, oder gastro-beständige Kapsel nach dem Anspruch 5 oder 6, für deren Verwendung bei der Behandlung einer Krankheit oder einer Störung in Verbindung mit einem Ungleichgewicht der Darm-Biozönose, ausgewählt aus der Colitis mit *Clostridium difficile* und deren Rezidive, den chronischen entzündlichen Erkrankungen des Darms, wie Morbus Crohn und Colitis ulcerosa, dem Reizdarmsyndrom, dem Tragen multiresistenter Bakterien, der Verstopfung, dem Reisedurchfall, den Infektionen des Verdauungstrakts, den Autoimmunkrankheiten, der Divertikulose, der Diabetes des Typs 1 und des Typs 2, dem metabolischen Syndrom, der Fettleibigkeit, der rheumatischen Arthritis, der Spondylitis ankylosans und Sakroiliitis, dem chronischen Erschöpfungssyndrom, der Halitosis, der Allergie, der Akne, der Schlaflosigkeit, den Depressionen und den neuropsychiatrischen Erkrankungen, wie der Parkinson-Krankheit und den Krankheiten des Autismus-Spektrums.

8. Lyophilisierte Zusammensetzung oder gastro-beständige Kapsel für deren Verwendung nach Anspruch 7, im Anschluss an eine orale Antibiotikabehandlung im Fall von Rezidiven von Infektionen mit *Clostridium difficile.*

9. Verfahren zur Herstellung einer gastro-beständigen Kapsel wie im Anspruch 5 oder 6 definiert, die folgenden Schritte umfassend:
a. Erfassung einer Biozönose in ihrem Ökosystem;
b. Verdünnung der Biozönose in ihrem Ökosystem mit einem Gemisch an Kryoprotektoren, umfassend Maltodextrin und Trehalose in einem Gewichtsverhältnis M/T zwischen 35/65 und 45/65;
c. Lyophilisieren des im Schritt b erhaltenen Gemisches;
d. Zerstäuben des in Schritt c erhaltenen Lyophilisats;
e. Eventuell Beigeben von einem oder mehreren Hilfsstoffen zur Erleichterung des Flusses des Lyophilisats;
f. Füllen der Hülle der gastro-beständigen Kapsel mit dem lyophilisierten Gemisch;
g. Schließen der Kapseln,
wobei die Biozönose in ihrem Ökosystem die Darm-Biozönose, die Fäkal-Biozönose oder die vaginale Biozönose ist.

10. Verfahren nach Anspruch 9, wobei die Biozönose in ihrem Ökosystem aus Stühlen besteht, und das Gemisch von Kryoprotektoren aus einem Gemisch aus Maltodextrin (M) und Trehalose (T) in einem Gewichtsverhältnis M/T von 40/60 besteht.

## Claims

1. Use of a mixture of cryoprotectants for preserving a microbiota in its ecosystem in lyophilised form, **characterised in that** said mixture of cryoprotectants comprises maltodextrin (M) and trehalose (T) in an M/T weight ratio of between 35/65 and 45/55, the microbiota in its ecosystem being intestinal microbiota, faecal microbiota or vaginal microbiota.

2. Use according to claim 1, wherein the microbiota in its ecosystem consists of stools.

3. Lyophilised composition comprising a microbiota in its ecosystem and a mixture of cryoprotectants, **characterised in that** said mixture of cryoprotectants comprises maltodextrin and trehalose, used in an M/T weight ratio of between 35/65 and 45/55, the microbiota in its ecosystem being intestinal microbiota, faecal microbiota or vaginal microbiota.

4. Lyophilised composition according to claim 3, wherein (i) the microbiota in its ecosystem (MDSE) consists of stools, (ii) the quantity by weight of stools represents 15 to 35% of the lyophilised composition and the quantity by weight of the mixture of cryoprotectants represents 85 to 65% of the lyophilised composition and (iii) maltodextrin (M) and trehalose (T) are used in an M/T weight ratio of 40/60.

5. Gastro-resistant capsule comprising a lyophilised composition such as defined in one of claims 3 or 4.

6. Gastro-resistant capsule according to claim 5, **characterised in that** said lyophilised composition is mixed with at least one flow excipient.

7. Lyophilised composition according to one of claims 3 or 4 or gastro-resistant capsule according to claim 5 or 6, for its use in the treatment of a disease or a disorder linked to an imbalance of intestinal microbiota selected from *Clostridium difficile* colitis and its recurrences, chronic inflammatory diseases of the intestine such as Crohn's disease and haemorrhagic rectocolitis, irritable bowel syndrome, the carrying of multi-resistant bacteria, constipation, traveller's diarrhoea, digestive infections, autoimmune diseases, diverticulosis, type 1 and type 2 diabetes, metabolic syndrome, obesity, rheumatoid polyarthritis, ankylosing spondylitis and sacroiliitis, chronic fatigue syndrome, halitosis, allergies, acne, insomnia, depression and neuropsychiatric diseases such as Parkinson's disease and illnesses on the autism spectrum.

8. Lyophilised composition or gastro-resistant capsule for its use according to claim 7, as a back-up to oral antibiotic treatment, in case of *Clostridium difficile* infection recurrences.

9. Method for preparing a gastro-resistant capsule such as defined in claim 5 or 6, comprising the following steps:
a. collecting a microbiota in its ecosystem;
b. dilution of said microbiota in its ecosystem with a mixture of cryoprotectants comprising maltodextrin and trehalose in an M/T weight ratio of between 35/65 and 45/65;
c. lyophilisation of the mixture obtained in step b;
d. spraying of the lyophilizate obtained in step c;
e. possibly adding one or more excipients facilitating the flow of the lyophilizate;
f. filling of the case of the gastro-resistant capsule with said lyophilised mixture;
g. closing of the capsules,
the microbiota in its ecosystem being intestinal microbiota, faecal microbiota or vaginal microbiota.

10. Method according to claim 9, wherein said microbiota in its ecosystem consists of stools, and the mixture of cryoprotectants consists of a mixture of maltodextrin (M) and trehalose (T) in an M/T weight ratio of 40/60.
